(19) Europäisches Patentamt
European Patent Office
Office européen des brevets

(11) Numéro de publication: 0 251 859 B1

# (12) FASCICULE DE BREVET EUROPEEN

(45) Date de publication du fascicule du brevet: 22.11.90

(51) Int. Cl.⁵: **C07D 471/04**, C07C 235/06 // (C07D471/04, 235:00, 221:00)

(21) Numéro de dépôt: 87401353.5

(22) Date de dépôt: 17.06.87

## (54) Procédé de préparation d'imidazopyridines.

(30) Priorité: 27.06.86 FR 8609330

(43) Date de publication de la demande: 07.01.88 Bulletin 88/1

(45) Mention de la délivrance du brevet: 22.11.90 Bulletin 90/47

(84) Etats contractants désignés: AT BE CH DE ES FR GB GR IT LI LU NL SE

(56) Documents cités:
EP-A- 0 022 626
EP-A- 0 050 563

BULLETIN DES SOCIETES CHIMIQUES BELGES, vol. 86, no. 11, 1977, pages 879-885, Bruxelles, BE; R. VERHE et al.: "Reactions of N-1-(2,2,2-trichloroethylidene)t. butylamine with nucleophilic reagents"
CHEMISCHE BERICHTE, vol. 104, 1971, pages 3475-3485, Weinheim, DE; H. BREDERECK et al.: "Darstellung und Reaktionen substituierter Amidacetale"
CHEMISCHE BERICHTE, vol. 100, 1967, pages 2292-2295, Weinheim, DE; K. SCHANK: "Sekundäre Dialkoxy-acetamide aus Diketen"

(73) Titulaire: SYNTHELABO, 58 rue de la Glacière, F-75013 Paris(FR)

(72) Inventeur: Rossey, Guy, 8, Square Lebrun Voisins-le-Bretonneux, F-78180 Montigny le Bretonneux(FR)
Inventeur: Long, David, Résidence les Prés 9, Allée des Romarins, F-78180 Montigny le Bretonneux(FR)

(74) Mandataire: Thouret-Lemaitre, Elisabeth et al, SYNTHELABO Service Brevets 22, avenue Galilée, F-92352 LE PLESSIS ROBINSON CEDEX(FR)

(56) Documents cités: (suite)
CHEMICAL ABSTRACTS, vol. 91, page 606, 1979, résumé no. 55805a, Columbus, Ohio, US; M. MEYER ZUR HEYDE: "New applications of trichloroacetylisocyanate in proton NMR spectroscopy", & FRESENIUS' Z. ANAL. CHEM. 1979, 295(2-3), 125-42

EP 0 251 859 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé de préparation d'imidazopyridines qui répondent à la formule (I) donnée en annexe dans laquelle
Y représente un atome d'hydrogène ou d'halogène ou un radical $(C_{1-4})$alkyle,
$X_1$ et $X_2$ sont chacun, indépendamment l'un de l'utre, un atome d'hydrogène ou d'halogène, un radical $(C_{1-4})$alcoxy, un radical $(C_{1-6})$alkyle, le groupe $CF_3$, $CH_3S$, $CH_3SO_2$ ou $NO_2$ et
$R_1$ et $R_2$ représentent chacun indépendamment l'un de l'autre soit un atome d'hydrogène, soit un radical $(C_{1-5})$alkyle droit ou ramifié, $R_1$ et $R_2$ ne pouvant être tous deux des atomes d'hydrogène.

Ces composés sont décrits dans le brevet européen n° 0050563 de la demanderesse.

Le procédé de l'invention, décrit en annexe, consiste à faire réagir une imidazopyridine de formule (II) avec un composé (III) à une température de 20 à 100°C, soit dans un solvant en présence d'un acide, par exemple un solvant chloré tel que le dichloroéthane, ou le dichlorométhane, soit dans un acide tel que l'acide formique, acétique préférentiellement, propionique ou butyrique puis à faire réagir le composé obtenu (IV) avec du chlorure de thionyle, du chlorure de phosphoryle, du phosgène ou du chlorure d'oxalyle, dans un solvant tel que le dichlorométhane, le dichloroéthane ou tout solvant chloré et enfin à faire réagir le composé (V) avec un agent réducteur, tel que $NaBH_4$ , $Zn(BH_4)_2$, $KBH_4$, $LiBH_4$ dithionite ou leurs dérivés, Zn/HCl de manière à obtenir le composé (I) que l'on peut transformer en sel.

Le procédé de l'invention permet d'obtenir les composés (I) avec un excellent rendement allant de 50 à 95 % et dans des conditions excellentes de pureté.

Les composés de départ (III) et intermédiaires de formules (IV) et (V) données en annexe sont nouveaux et font partie de l'invention.

Les composés de départ (II) sont décrits dans la littérature. Les composés de départ (III) dans lesquels $R_1$ et $R_2$ représentent chacun indépendamment l'un de l'autre soit un atome d'hydrogène, soit un radical $(C_{1-5})$alkyle droit ou ramifié, $R_1$ et $R_2$ ne pouvant être tous deux des atomes d'hydrogène, et $R_3$ représente un radical $(C_{1-4})$alkyle, sont nouveaux et peuvent être préparés à partir des compsés correspondants de formule

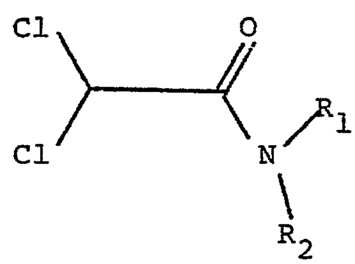

dans laquelle $R_1$ et $R_2$ ont les mêmes significations que ci-dessus, par réaction avec un alcanolate de sodium et/ou un alcanol dans de l'acétonitrile.

Les composés intermédiaires (IV) et (V) sont obtenus selon le procédé dont le schéma est donné dans l'annexe.

Exemple 1. Préparation du composé de départ (III) : N,N-diméthyl-diméthoxy-2,2 acétamide.

1.1 N,N-diméthyl dichloro-2,2 acétamide

Dans un réacteur à double enveloppe on introduit 550 ml (5 moles), de diméthylamine à 40 % dans de l'eau, puis on refroidit la double enveloppe à environ -10°C. On ajoute ensuite, goutte à goutte, en 4 h, 192,4 ml (2 moles) du chlorure de l'acide dichloro-2,2 acétique. On contrôle la température de la réaction entre -10 et 0°C. Lorsque la réaction est terminée on laisse décanter, dans le réacteur, le produit qui est plus dense que l'eau. On récupère ce produit et extrait la phase aqueuse au dichlorométhane. On mélange les phases organiques. On les lave pour éliminer l'acide.
Eb : 109°C sous 15 mmHg.

1.2. N,N-diméthyl diméthoxy-2,2 acétamide.

Dans un ballon de 500 ml on introduit 73,6 g du composé obtenu précédemment puis on ajoute 150 ml d'acétonitrile ($CH_3CN$). On ajoute ensuite assez rapidement 180 ml (2 équivalents) de méthylate de sodium à 29 % dans du méthanol. On chauffe à la température de reflux pendant 3 h. On laisse refroidir. On filtre le chlorure de sodium, on évapore les solvants puis reprend (3 fois) avec 50 ml de t-butyl-méthyl-éther (TBME) pour éliminer le chlorure de sodium restant. On reprend le mélange avec 100 ml d'eau et

laisse décanter. La phase aqueuse est évaporée à sec puis reprise avec du TBME, le chlorure de sodium est filtré. On groupe les phases organiques puis on évapore les solvants. On obtient alors le produit.
Eb = 101°C (15 mmHg).

Exemple 2. N,N-di-n-propyl diméthoxy-2,2 acétamide.

2.1. N,N-di-n-propyl dichloro-2,2 acétamide.

Dans un erlenmeyer de 500 ml on fait réagir 60 g de dipropylamine avec 40,4 g de chlorure de dichloracétyle à 0°C dans 100 ml de dichlorométhane. La solution jaune est lavée par 200 ml d'eau, séchée et concentrée. On obtient une huile qui cristallise.
F = 53°C.

2.2. N,N-di-n-propyl diméthoxy-2,2 acétamide.

On fait réagir 160 g de méthylate de sodium avec 66,4 g du composé obtenu ci-dessus dans 300 ml d'acétonitrile,à 80°C, pendant 50 minutes. On refroidit et acidifie le mélange réactionnel par HCl, filtre les sels, concentre, reprend le résidu par 300 ml de dichlorométhane. La phase organique est lavée à l'eau, séchée et concentrée. On obtient une huile.
Eb = 140°C (15 mmHg).

Exemple 3. Méthyl-6 N,N-diméthyl (méthyl-4 phényl)-2 imidazo [1,2-ª]pyridine-3-acétamide.

3.1. Méthyl-6 N,N-diméthyl (méthyl-4 phényl)-2 α-hydroxy imidazo[1,2-ª]pyridine-3-acétamide (composé IV).

Dans un réacteur de 2 litres, équipé pour la distillation azéotropique, on introduit 93 g de composé (III) dans lequel $R_1$, $R_2$ et $R_3$ représentent chacun le radical méthyle, 15,8 ml d'eau, 64,8 ml d'acide acétique concentré et 15,8 ml d'acide chlorhydrique à 37 %. On chauffe le mélange vers 43-46°C pendant 50 mn.
On ajuste alors le pH à 4-5 par 17 g d'acétate de sodium et ajoute 850 ml de dichloro-1,2 éthane et 100 g de méthyl-6 (méthyl-4 phényl)-2 imidazo[1,2-ª]pyridine.
On porte alors le mélange à reflux pendant 2 heures. Pendant ce temps, on élimine environ 20 ml d'eau par azétrope (température de l'azéotropie : 78°C).
On ramène la température à 73°C et on ajoute, goutte à goutte, 250 ml d'une solution saturée de bicarbonate de sodium, puis 35 ml de lessive de soude de façon à ajuster le pH à 7.
On élimine alors le solvant par distillation, puis refroidit à 55°C et élimine sous vide les dernières traces de dichloro-1,2 éthane.
On ajoute 55 ml d'eau, 250 ml d'isopropanol et de lessive de soude pour atteindre pH 10, ce qui entraîne la cristallisation du produit sous forme d'un solide blanc.
Le produit est essoré, lavé à l'eau et séché sous vide en présence de $P_2O_5$ pendant 6 h.
Le rendement est de 89 %.
F = 174-176°C.

3.2. Chlorhydrate de méthyl-6 N,N-diméthyl (méthyl-4 phényl)-2 α-chloro imidazo[1,2-ª]pyridine-3-acétamide (composé V).

Dans un réacteur de 0,5 1, on introduit 40 g du composé (IV) obtenu précédemment et 180 ml de dichloro-1,2 éthane.
On chauffe le mélange à 50°C et introduit, goutte à goutte, une solution de 11,2 ml de chlorure de thionyle dans 30 ml de dichloro-1,2 éthane en 1 h.
La température s'élève à 60°C.
On chauffe alors à reflux pendant 1 h 30, refroidit vers 40-50°C et évapore sous vide une partie du dichloro-1,2 éthane pour chasser l'excès de $SOCl_2$.
On ajoute alors 200 ml d'éther isopropylique et refroidit à 10°C puis agite pendant 1 h 30.
On rince le solide par 100 ml d'éther isopropylique et le sèche sous vide, en étuve, à 50-60°C pendant 6 h.
Le rendement est de 97,6 %.
F = 186°C (dec).

3.3. Méthyl-6 N,N-diméthyl (méthyl-4 phényl)-2 imidazo[1,2-ª]pyridine-3-acétamide et son hémitartrate (composé I).

3.3.1. Dans un réacteur de 1 litre, on dissout 50 g du chlorhydrate obtenu sous 2 dans 250 ml de méthanol.

On ajoute alors rapidement en 2-3 mn, une solution de 20 g de borohydrure de sodium dans 150 ml d'eau.
On maintient l'agitation pendant 1 h 30.
On ajoute alors 150 ml de solution saturée de carbonate de sodium et 250 ml d'eau pour achever la précipitation.
Le précipidé brun-clair est essoré, lavé abondamment à l'eau et séché en étuve pendant 18 h.
Le rendement est de 62 %. F = 194-196°C.

3.3.2. On dissout 25 g du composé (I) dans 180 ml de méthanol.
On ajoute une solution de 6,1 g d'acide L (+) tartrique dans 60 ml de méthanol.
On laisse cristalliser.
Le rendement est de 94 %. F = 197°C.

Exemple 4. N,N-di-n-propyl chloro-6 (chloro-4 phényl)-2 imidazo[1,2-a]pyridine-3-acétamide.

4.1. N,N-di-n-propyl chloro-6 (chloro-4 phényl)-2 α-hydroxy imidazo[1,2-a]pyridine-3-acétamide.

On agite un mélange de 56,5 ml (0,226 mol) de composé (III) dans lequel $R_1$ et $R_2$ sont des radicaux n-propyle et $R_3$ est le radical méthyle, 67 ml d'eau, 28 ml d'acide acétique, 67 ml d'acide chlorhydrique concentré (12N), à 50°C, pendant 20 mn.
On ajoute alors 83,3 g (1 mole) d'acétate de sodium et agite 30 mn. On ajoute alors 50 g (0,19 mole) de chloro-6 (chloro-4 phényl)-2 d'imidazo[1,2-a]pyridine et on agite le mélange réactionnel à 90°C pendant 2 h.
Après refroidissement à 20°C, on fait précipiter le produit à pH 10 par addition d'environ 100 ml de lessive de soude 10N, après 16 h d'agitation à la température ambiante. Le précipité est filtré, lavé 4 fois par 100 ml d'eau et séché en étuve à vide (70°C, 20 mbar). Le rendement est de 93,8 %.
F = 133°C.

4.2. Chlorhydrate de chloro-6 N,N-di-n-propyl (chloro-4 phényl)-2 α-chloro[imidazo-1,2-a]pyridine-3-acétamide.

A une solution de 21,6 ml (0,3 mole) de chlorure de thionyle dans 100ml de dichloro-1,2 éthane on ajoute une suspension de 100 g (0,237 mol) du composé précédemment obtenu dans 340 ml de dichloro-1,2 éthane.
On agite le mélange pendant 16 h à la température ambiante. On le chauffe alors et on maintient à la température de70°C pendant 1 h jusqu'à ce que le dégagement gazeux cesse. On élimine alors 140 ml de solvant sous pression réduite.
En refroidissant à 10°C, on ajoute 340 ml d'éther diisopropylique pour parfaire la précipitation. Après une heure d'agitation à cette température, le précépité est filtré, lavé deux fois par 100 ml d'éther diisopropylique et séché sous vide (60°C, 10 mbar) pendant 8 h. Le rendement est de 95 %.
F = 190°C (dec).

4.3. N,N-di-n-propyl chloro-6 (chloro-4 phényl)-2 imidazo[1,2-a]pyridine-3-acétamide.

On ajoute à une suspension de 50 g (0,105 mol) du composé obtenu précédemment dans 300 ml d'isopropanol, en 22 mn, une solution de 17 g (0,37 mol) de borohydrure de sodium ($NaBH_4$) dans 400 ml d'eau en maintenant la température voisine de 20°C, par circulation de glycol à -5°C dans la double enveloppe du réacteur. Après 1 h d'agitation, on ajoute 300 ml d'eau, on agite de nouveau 1 h et on filtre le solide qui est lavé 8 fois par 150 ml d'eau et 3 fois par 70 ml d'éther diisopropylique et séché en étuve pendant 8 h (60°C, 10 mbar). Le rendement est de 50 %.
F = 138-139°C. (polymorphe B).

D'autres composés (I) peuvent être préparés selon le même schéma réactionnel.

Les composés (III), (IV) et (V) préparés à titre d'exemples, sont représentés dans les tableaux suivants :

Tableau (I)

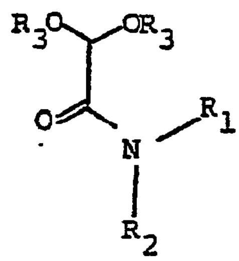

(III)

| Composé | $R_1$ | $R_2$ | $R_3$ | Eb(°C) |
|---|---|---|---|---|
| 1 | $CH_3$ | $CH_3$ | $CH_3$ | 101 (15 mmHg) |
| 2 | $n-C_3H_7$ | $n-C_3H_7$ | $CH_3$ | 140 (15 mmHg) |
| 3 | $CH_3$ | $CH_3$ | $C_2H_5$ | 105 (14 mmHg) |
| 4 | $n-C_3H_7$ | $n-C_3H_7$ | $C_2H_5$ | 135 (13 mmHg) |

Tableau (II)

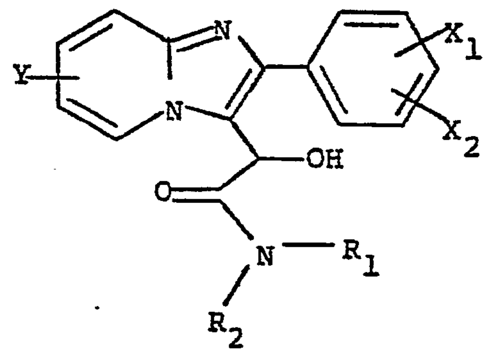

(IV)

| Composé | Y | $X_1$ | $X_2$ | $R_1$ | $R_2$ | F(°C) |
|---|---|---|---|---|---|---|
| 1 | 6-$CH_3$ | 4-Cl | H | $CH_3$ | $CH_3$ | 234 |
| 2 | 6-Cl | 4-Cl | H | n-$C_3H_7$ | n-$C_3H_7$ | 130-132 |
| 3 | 8-$CH_3$ | 4-Cl | H | $CH_3$ | $CH_3$ | 191-192 |
| 4 | 6-$CH_3$ | 4-$CH_3$ | H | $CH_3$ | $CH_3$ | 174-175 |
| 5 | 6-$CH_3$ | 3-$CH_3$ | H | $CH_3$ | $CH_3$ | 191-192 |

Tableau (III)

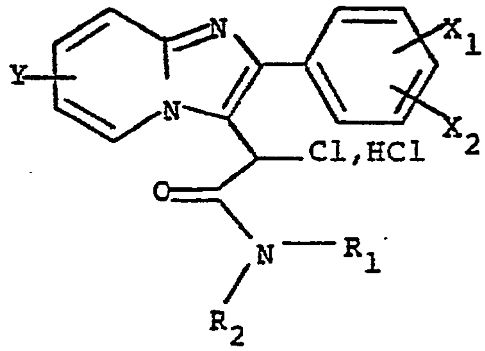

(V)

| Composé | Y | $X_1$ | $X_2$ | $R_1$ | $R_2$ | F(°C) |
|---|---|---|---|---|---|---|
| 1 | 6-$CH_3$ | 4-$CH_3$ | H | $CH_3$ | $CH_3$ | 186(dec) |
| 2 | 6-Cl | 4-Cl | H | n-$C_3H_7$ | n-$C_3H_7$ | 190(dec) |

ANNEXE

Schéma réactionnel

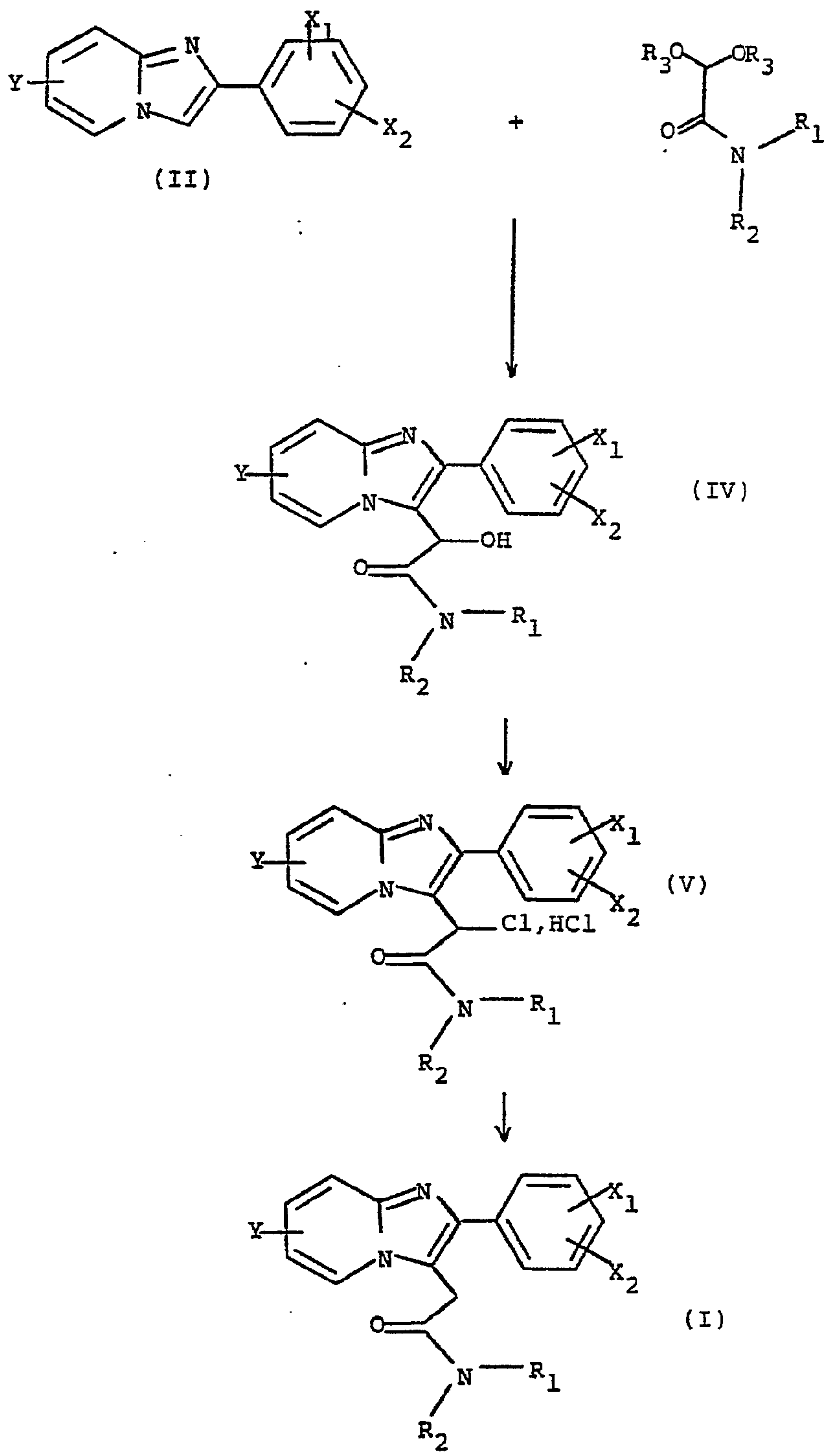

(II) + (III)

(IV)

(V)

(I)

**Revendications pour les Etats contractants: BE, CH, DE, FR, GB, IT, LI, LU, NL, SE**

1. Procédé de préparation d'imidazopyridines répondant à la formule (I)

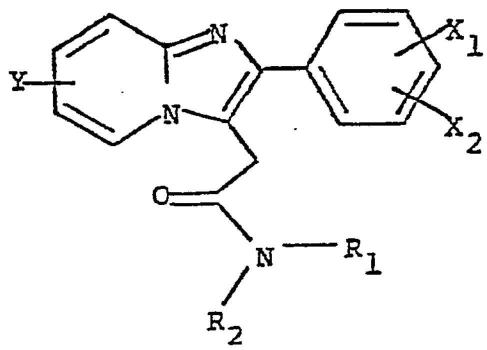

(I)

dans laquelle
Y représente un atome d'hydrogène ou d'halogène ou un radical $(C_{1-4})$alkyle,
$X_1$ et $X_2$ sont chacun, indépendamment l'un de l'autre, un atome d'hydrogène ou d'halogène, un radical $(C_{1-4})$alcoxy, un radical $(C_{1-6})$alkyle, le groupe $CF_3$, $CH_3S$, $CH_3SO_2$ ou $NO_2$ et
$R_1$ et $R_2$ représentent chacun, indépendamment l'un de l'autre, soit un atome d'hydrogène, soit un radical $(C_{1-5})$alkyle droit ou ramifié, $R_1$ et $R_2$ ne pouvant être tous deux des atomes d'hydrogène,
procédé caractérisé en ce que l'on fait réagir une imidazopyridine de formule (II)

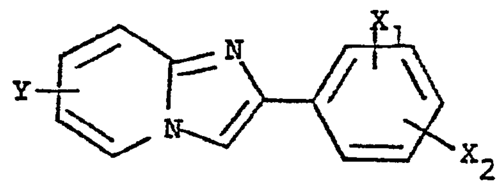

(II)

avec un composé de formule (III)

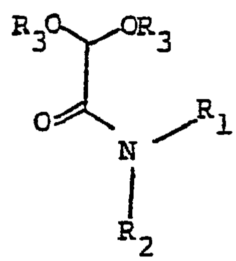

(III)

dans lequel $R_3$ est un radical $(C_{1-4})$alkyle, les autres radicaux Y, $X_1$, $X_2$, $R_1$ et $R_2$ ayant les significations données ci-dessus, à une température de 20 à 100°C, dans un solvant ou dans un acide, puis on fait réagir le composé de formule (IV)

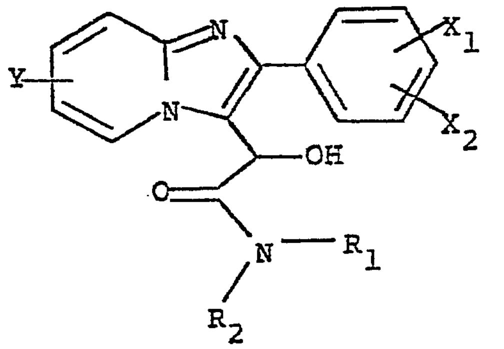

(IV)

avec un composé libérant du chlore, dans un solvant chloré, et enfin on fait réagir le composé de formule (V)

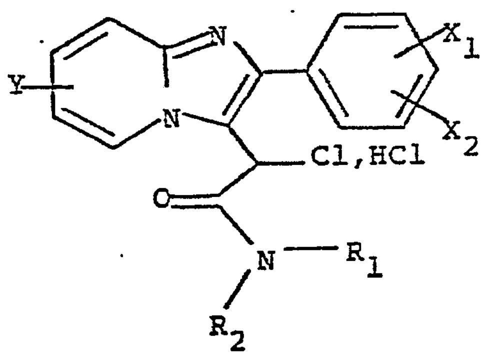

(V)

avec un agent réducteur pour obtenir le composé (I) que l'on peut transformer en sel.

2. Procédé selon la revendication 1, caractérisé par le fait que l'agent réducteur est $NaBH_4$, $LiBH_4$, $Zn(BH_4)_2$, un dithionite ou leurs dérivés ou le mélange Zn/HCl.

3. Procédé selon la revendication 1, caractérisé par le fait que le composé libérant du chlore est le chlorure de thionyle, le chlorure de phosphoryle, le phosgène ou le chlorure d'oxalyle.

4. Composés répondant à la formule (IV)

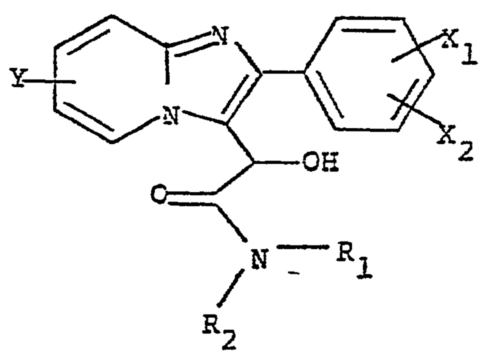

(IV)

dans lequelle
Y représente un atome d'hydrogène ou d'halogène ou un radical $(C_{1-4})$alkyle,
$X_1$ et $X_2$ sont chacun, indépendamment l'un de l'autre, un atome d'hydrogène ou d'halogène, un radical $(C_{1-4})$alcoxy, un radical $(C_{1-6})$alkyle, le groupe $CF_3$, $CH_3S$, $CH_3SO_2$ ou $NO_2$ et
$R_1$ et $R_2$ représentent chacun, indépendamment l'un de l'autre, soit un atome d'hydrogène, soit un radical $(C_{1-5})$alkyle droit ou ramifié, $R_1$ et $R_2$ ne pouvant être tous deux des atomes d'hydrogène.

5. Composés répondant à la formule (V)

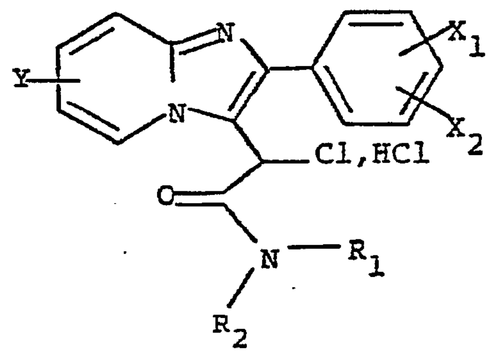

(V)

dans laquelle
Y représente un atome d'hydrogène ou d'halogène ou un radical $(C_{1-4})$alkyle,
$X_1$ et $X_2$ sont chacun, indépendamment l'un de l'autre, un atome d'hydrogène ou d'halogène, un radical $(C_{1-4})$alcoxy, un radical $(C_{1-6})$alkyle, le groupe $CF_3$, $CH_3S$, $CH_3SO_2$ ou $NO_2$ et
$R_1$ et $R_2$ représentent chacun, indépendamment l'un de l'autre, soit un atome d'hydrogène, soit un radical $(C_{1-5})$alkyle droit ou ramifié, $R_1$ et $R_2$ ne pouvant être tous deux des atomes d'hydrogène.

**Revendications pour les Etats contractants: AT, ES, GR**

1. Procédé de préparation d'imidazopyridines répondant à la formule (I)

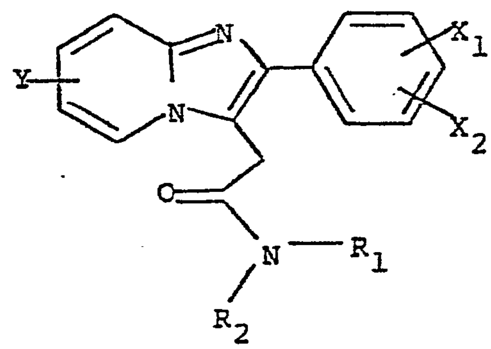

(I)

dans laquelle
Y représente un atome d'hydrogène ou d'halogène ou un radical $(C_{1-4})$alkyle,
$X_1$ et $X_2$ sont chacun, indépendamment l'un de l'autre, un atome d'hydrogène ou d'halogène, un radical $(C_{1-4})$alcoxy, un radical $(C_{1-6})$alkyle, le groupe $CF_3$, $CH_3S$, $CH_3SO_2$ ou $NO_2$ et
$R_1$ et $R_2$ représentent chacun, indépendamment l'un de l'autre, soit un atome d'hydrogène, soit un radical $(C_{1-5})$alkyle droit ou ramifié, $R_1$ et $R_2$ ne pouvant être tous deux des atomes d'hydrogène,
procéde caractérisé en ce que l'on fait réagir une imidazopyridine de formule (II)

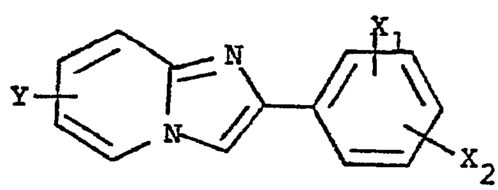

(II)

avec un composé de formule (III)

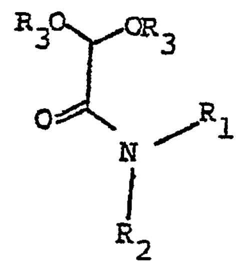

(III)

dans lequel $R_3$ est un radical $(C_{1-4})$alkyle, les autres radicaux Y, $X_1$, $X_2$, $R_1$ et $R_2$ ayant les significations données ci-dessus, à une température de 20 à 100°C, dans un solvant ou dans un acide, puis on fait réagir le composé de formule (IV)

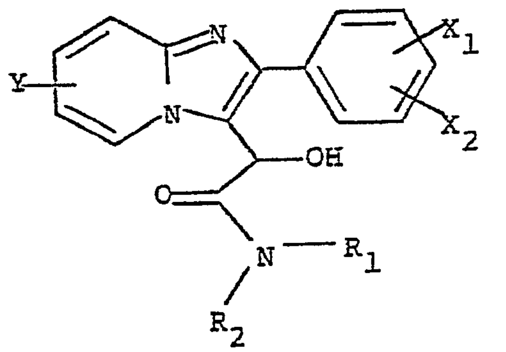

(IV)

avec un composé libérant du chlore, dans un solvant chloré, et enfin on fait réagir le composé de formule (V)

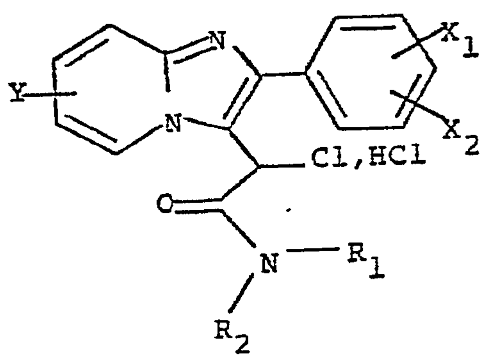

(V)

avec un agent réducteur pour obtenir le composé (I) que l'on peut transformer en sel.

2. Procédé selon la revendication 1, caractérisé par le fait que l'agent réducteur est $NaBH_4$, $LiBH_4$, $Zn(BH_4)_2$, un dithionite ou leurs dérivés ou le mélange Zn/HCl.

3. Procédé selon la revendication 1, caractérisé par le fait que le composé libérant du chlore est le chlorure de thionyle, le chlorure de phosphoryle, le phosgène ou le chlorure d'oxalyle.

**Claims for the Contracting States: BE, CH, DE, FR, GB, IT, LI, LU, NL, SE**

1. Process for the preparation of imidazopyridines corresponding to the formula (I)

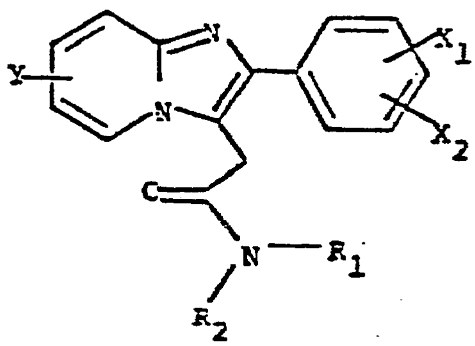

(I)

in which
Y denotes a hydrogen or halogen atom or a $C_{1-4}$ alkyl radical, each of
$X_1$ and $X_2$ is, independently of each other, a hydrogen or halogen atom, a $C_{1-4}$ alkoxy radical, a $C_{1-6}$ alkyl radical or the $CF_3$, $CH_3S$, $CH_3SO_2$ or $NO_2$ group, and each of
$R_1$ and $R_2$ denotes, independently of each other, either a hydrogen atom or a straight or branched $C_{1-5}$ alkyl radical, $R_1$ and $R_2$ being incapable of both being hydrogen atoms,
the process characterized in that an imidazopyridine of formula (II)

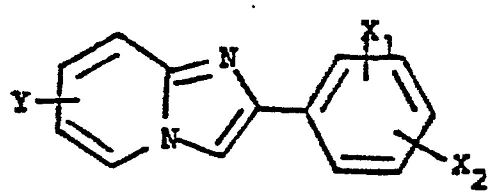

(II)

is reacted with a compound of formula (III)

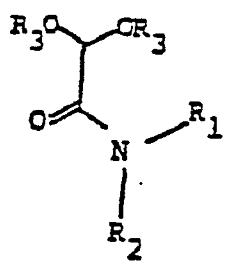

(III)

in which $R_3$ is a $C_{1-4}$ alkyl radical, the other radicals Y, $X_1$, $X_2$, $R_1$ and $R_2$ having the meanings given above, at a temperature of 20 to 100°C in a solvent or in an acid, and the compound of formula (IV)

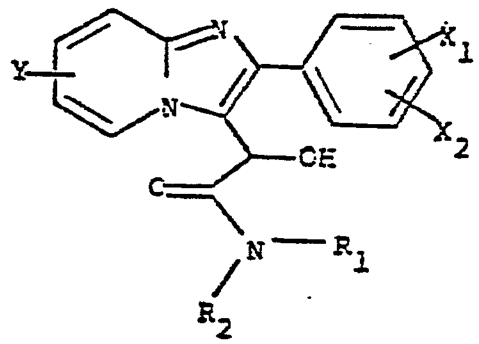

(IV)

is then reacted with a compound releasing chlorine, in a chlorinated solvent, and lastly the compound of formula (V)

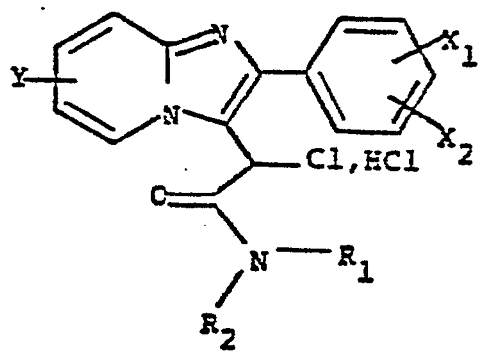

(V)

is reacted with a reducing agent to produce the compound (I) which may be converted into a salt.

2. Process according to claim 1, characterized in that the reducing agent is $NaBH_4$, $LiBH_4$, $Zn(BH_4)_2$, a dithionite or their derivatives or the mixture Zn/HCl.

3. Process according to claim 1, characterized in that the compound releasing chlorine is thionyl chloride, phosphoryl chloride, phosgene or oxalyl chloride.

4. Compounds corresponding to formula (IV)

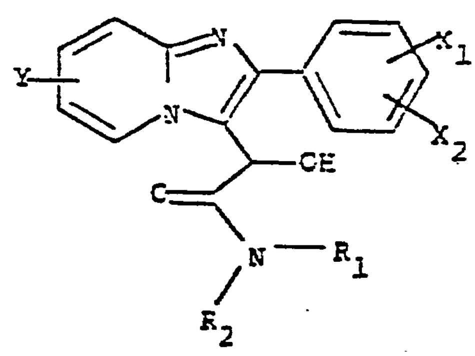

(IV)

in which
Y denotes a hydrogen or halogen atom or a $C_{1-4}$ alkyl radical, each of
$X_1$ and $X_2$ is, independently of each other, a hydrogen or halogen atom, a $C_{1-4}$ alkoxy radical, a $C_{1-6}$ alkyl radical or the $CF_3$, $CH_3S$, $CH_3SO_2$ or $NO_2$ group, and each of
$R_1$ and $R_2$ is, independently of each other, either a hydrogen atom or a straight or branched $C_{1-5}$ alkyl radical, $R_1$ and $R_2$ being incapable of both being hydrogen atoms.

5. Compounds corresponding to formula (V)

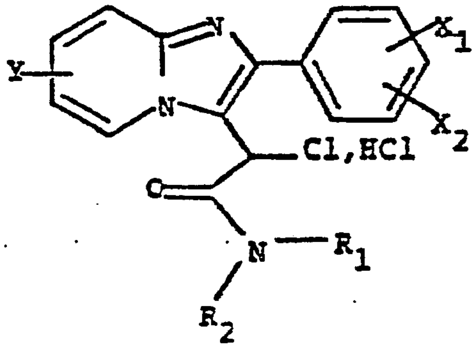

(V)

in which
Y denotes a hydrogen or halogen atom or a $C_{1-4}$ alkyl radical, each of
$X_1$ and $X_2$ is, independently of each other, a hydrogen or halogen atom, a $C_{1-4}$ alkoxy radical, a $C_{1-6}$ alkyl radical or the $CF_3$, $CH_3S$, $CH_3SO_2$ or $NO_2$ group, and each of
$R_1$ and $R_2$ denotes, independently of each other, either a hydrogen atom or a straight or branched $C_{1-5}$ alkyl radical, $R_1$ and $R_2$ being incapable of both being hydrogen atoms.

**Claims for the Contracting States: AT, ES, GR**

1. Process for the preparation of imidazopyridines corresponding to the formula (I)

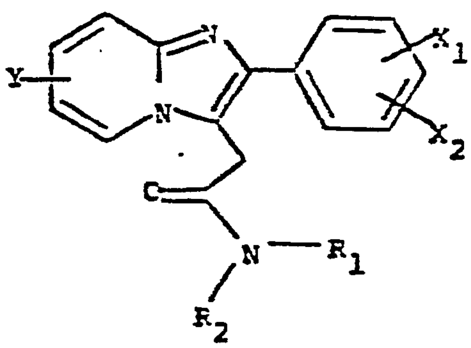

(I) (I)

in which
Y denotes a hydrogen or halogen atom or a $C_{1-4}$ alkyl radical, each of
$X_1$ and $X_2$ is, independently of each other, a hydrogen or halogen atom, a $C_{1-4}$ alkoxy radical, a $C_{1-5}$ alkyl radical or the $CF_3$, $CH_3S$ $CH_3SO_2$ or $NO_2$ group, and each of
$R_1$ and $R_2$ denotes, independently of each other, either a hydrogen atom or a straight or branched $C_{1-5}$ alkyl radical, $R_1$ and $R_2$ being incapable of both being hydrogen atoms, the process characterized in that an imidazopyridine of formula (II)

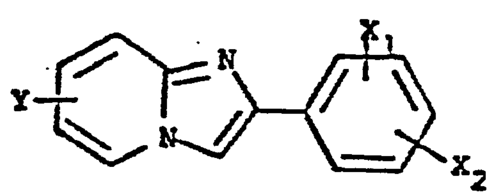

(II)

is reacted with a compound of formula (III)

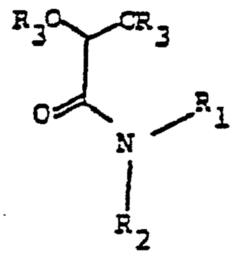

(III)

in which $R_3$ is a $C_{1-4}$ alkyl radical, the other radicals Y, $X_1$, $X_2$, $R_1$ and $R_2$ having the meanings given above, at a temperature of 20 to 100°C in a solvent or in an acid, and the compound of formula (IV)

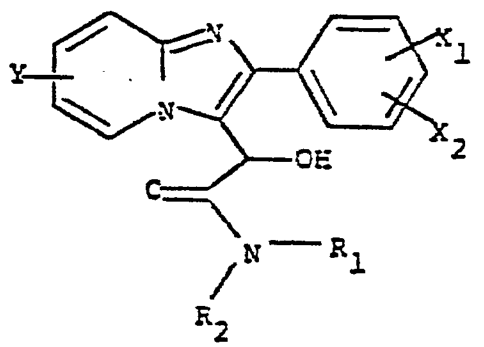

(IV)

is then reacted with a compound releasing chlorine, in a chlorinated solvent, and lastly the compound of formula (V)

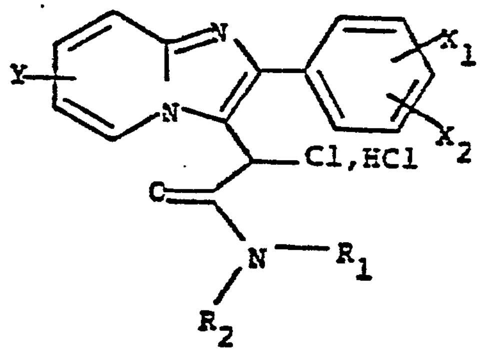

(V)

is reacted with a reducing agent to produce the compound (I) which may be converted into a salt.

2. Process according to claim 1, characterized in that the reducing agent is $NaBH_4$, $LiBH_4$, $Zn(BH_4)_2$, a dithionite or their derivatives or the mixture Zn/HCl.

3. Process according to claim 1, characterized in that the compound releasing chlorine is thionyl chloride, phosphoryl chloride, phosgene or oxalyl chloride.

**Patentansprüche für die Vertragsstaaten: BE, CH, DE, FR, GB, IT, LI, LU, NL, SE**

1. Verfahren zur Herstellung von Imidazopyridinen der Formel (I)

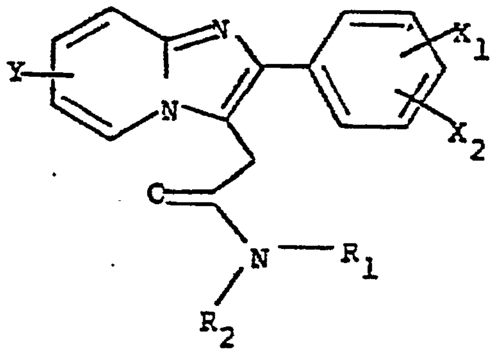

(I)

in welcher Y für ein Wasserstoff- oder ein Halogenatom oder für einen $(C_{1-4})$-Alkylrest steht, $X_1$ und $X_2$ jeweils unabhängig voneinander ein Wasserstoff- oder Halogenatom, einen $(C_{1-4})$-Alkoxy- oder $(C_{1-6})$-Alkylrest oder die Gruppe $CF_3$, $CH_3S$, $CH_3SO_2$ oder $NO_2$ bedeuten und $R_1$ und $R_2$ jeweils unabhängig voneinander für ein Wasserstoffatom oder einen geradkettigen oder verzweigten $(C_{1-5})$-Alkylrest stehen, wobei nicht beide Reste $R_1$ und $R_2$ ein Wasserstoffatom darstellen, dadurch gekennzeichnet, daß man ein Imidazopyridin der Formel (II)

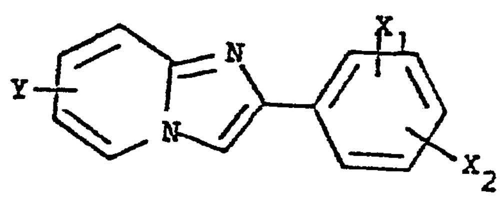

(II)

mit einer Verbindung der Formel (III)

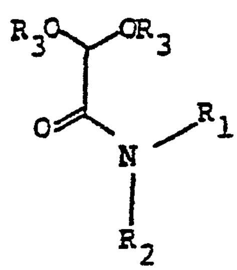

(III)

in welcher $R_3$ einen $(C_{1-4})$-Alkylrest bedeutet und die anderen Reste Y, $X_1$, $X_2$, $R_1$ und $R_2$ die oben genannte Bedeutung haben, bei einer Temperatur von 20 bis 100°C in einem Lösungsmittel oder in einer Säure reagieren läßt, dann die Verbindung der Formel (IV)

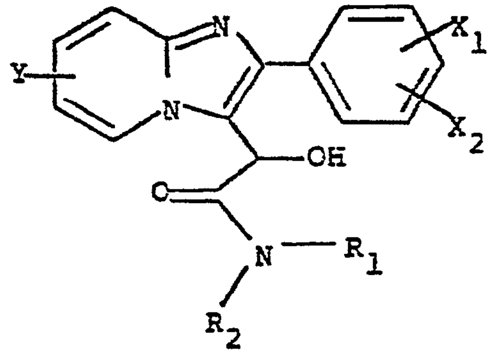

(IV)

mit einer Chlor freisetzenden Verbindung in einem chlorierten Lösungsmittel umsetzt und schließlich die Verbindung der Formel (V)

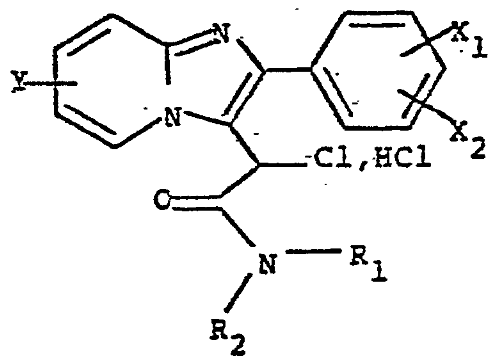

(V)

mit einem Reduktionsmittel umsetzt, um die Verbindung (I) zu erhalten, die man in ein Salz umwandeln kann.

2. Verfahren nach Anspruch 1, dadurch gekennzeichnet, daß das Reduktionsmittel $NaBH_4$, $LiBH_4$, $Zn(BH_4)_2$, ein Dithionit oder ein Derivat derselben oder die Mischung Zn/HCl ist.

3. Verfahren nach Anspruch 1, dadurch gekennzeichnet, daß die Chlor freisetzende Verbindung das Thionylchlorid, Phosphorylchlorid, Phosgen oder Oxalylchlorid ist.

4. Verbindungen der Formel (IV)

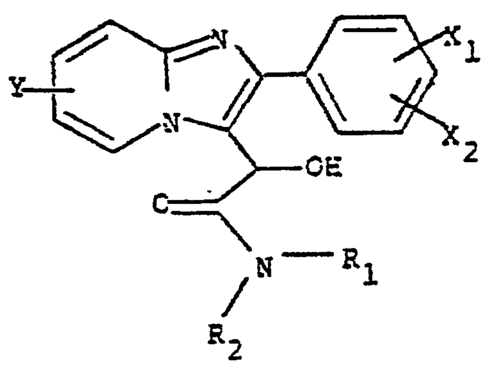

(IV)

in welcher Y für ein Wasserstoff- oder Halogenatom oder einen $(C_{1-4})$-Alkylrest steht, $X_1$ und $X_2$ jeweils unabhängig voneinander ein Wasserstoff- oder Halogenatom oder einen $(C_{1-4})$-Alkoxy-, $(C_{1-6})$-Alkylrest oder die Gruppe $CF_3$, $CH_3S$, $CH_3SO_2$ oder $NO_2$ bedeuten und $R_1$ und $R_2$ jeweils unabhängig voneinander für ein Wasserstoffatom oder einen geradkettigen oder verzweigten $(C_{1-5})$-Alkylrest stehen, wobei $R_1$ und $R_2$ nicht beide für ein Wasserstoffatom stehen.

5. Verbindungen der Formel (V)

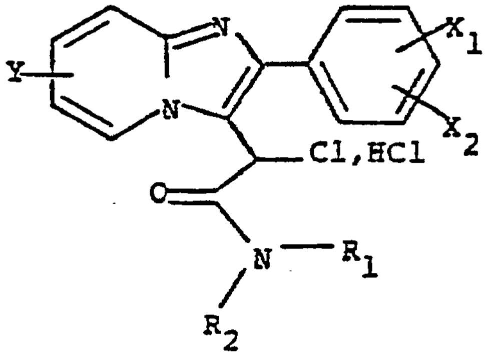

(V)

in welcher Y für ein Wasserstoff- oder Halogenatom oder einen $(C_{1-4})$-Alkylrest steht, $X_1$ und $X_2$ jeweils unabhängig voneinander für ein Wasserstoff- oder Halogenatom, einen $(C_{1-4})$-Alkoxy-, einen $(C_{1-6})$-Alkylrest oder die Gruppe $CF_3$, $CH_3S$, $CH_3SO_2$ oder $NO_2$ stehen und $R_1$ und $R_2$ unabhängig voneinander entweder ein Wasserstoffatom oder einen geradkettigen oder verzweigten $(C_{1-5})$-Alkylrest bedeuten, wobei $R_1$ und $R_2$ nicht beide für ein Wasserstoffatom stehen.

**Patentansprüche für die Vertragsstaaten: AT, ES, GR**

1. Verfahren zur Herstellung von Imidazopyridinen der Formel (I)

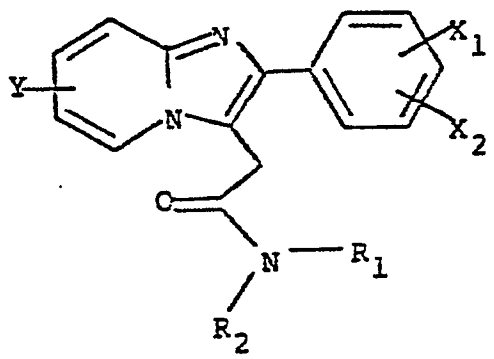

(I)

in welcher Y für ein Wasserstoff- oder Halogenatom oder für einen $(C_{1-4})$-Alkylrest steht, $X_1$ und $X_2$ jeweils unabhängig voneinander ein Wasserstoff- oder Halogenatom, einen $(C_{1-4})$-Alkoxy- oder $(C_{1-6})$-Alkylrest oder die Gruppe $CF_3$, $CH_3S$, $CH_3SO_2$ oder $NO_2$ bedeuten und $R_1$ und $R_2$ unabhängig voneinander für ein Wasserstoffatom oder einen geradkettigen oder verzweigten $(C_{1-5})$-Alkylrest stehen, wobei $R_1$ und $R_2$ nicht beide für Wasserstoffatome stehen, dadurch gekennzeichnet, daß man ein Imidazopyridin der Formel (II)

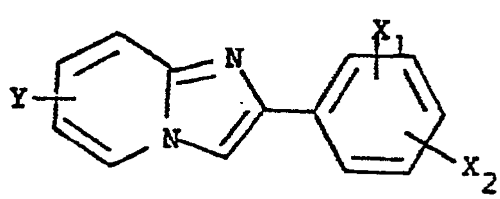

(II)

mit einer Verbindung der Formel (III)

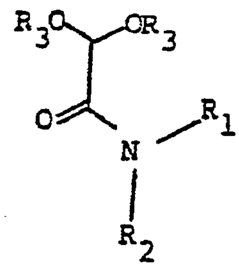

(III)

in welcher $R_3$ für einen $(C_{1-4})$-Alkylrest steht und die anderen Reste Y, $X_1$, $X_2$, $R_1$ und $R_2$ die oben genannte Bedeutung haben, bei einer Temperatur von 20 bis 100°C in einem Lösungsmittel oder einer Säure zur Reaktion bringt, dann die Verbindung der Formel (IV)

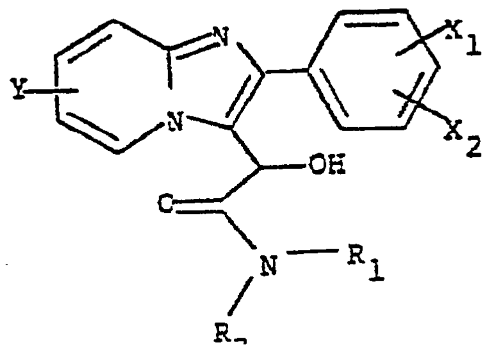

(IV)

mit einer Chlor in Freiheit setzenden Verbindung in einem chlorierten Lösungsmittel umsetzt und schließlich die Verbindung der Formel (V)

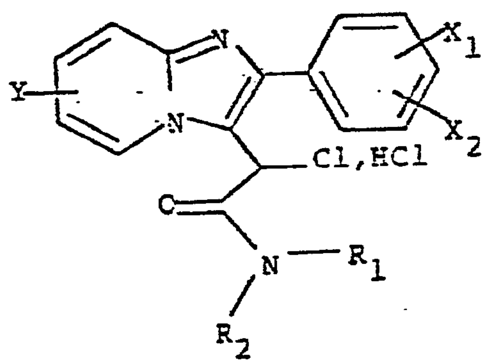

(V)

mit einem Reduktionsmittel zur Reaktion bringt, um die Verbindung (I) zu erhalten, die man in ein Salz umwandeln kann.

2. Verfahren nach Anspruch 1, dadurch gekennzeichnet, daß das Reduktionsmittel $NaBH_4$, $LiBH_4$, $Zn(BH_4)_2$, ein Dithionit oder ein Derivat derselben oder die Mischung Zn/HCl ist.

3. Verfahren nach Anspruch 1, dadurch gekennzeichnet, daß die Chlor freisetzende Verbindung das Thionylchlorid, Phosphorylchlorid, Phosgen oder Oxalylchlorid ist.